# EUROPEAN PATENT APPLICATION

(11) **EP 3 305 337 A1**
(43) Date of publication of application: **11.04.2018**
(21) Application number: 16803815.6
(22) Date of filing: 07.06.2016
(51) Int. Cl.: A61L 27/00, C12N 5/00

(54) **FUNCTIONAL CELL SHEET USING ELECTRICALLY ACTIVATED CONDUCTIVE POLYMER, AND METHOD OF PRODUCING SAME**

(30) Priority: 05.06.2015 KR 20150080206
(71) Applicant: National Cancer Center, Goyang-si, Gyeonggi-do 10408 (KR)
(72) Inventor: CHO, Young Nam, Goyang-si Gyeonggi-do 10234 (KR); LEE, Hyung Jae, Seoul 04736 (KR)
(74) Representative: Rigby, Barbara Nicole
(86) International application number: PCT/KR2016/006015
(87) International publication number: WO 2016/195462

(57) **Abstract**

The present invention relates to a functional cell sheet using an electroactive conductive polymer and a method of preparing the same, and more particularly, to a cell sheet for tissue engineering which is a growth factor-immobilized cell sheet formed in a single-layer or 3D multilayer form and a composition for inducing osteogenic differentiation including the same.

## Description

### [Technical Field]

The present invention relates to a functional cell sheet using an electro active conductive polymer and a method of preparing the same, and more particularly, to a cell sheet for tissue engineering which is a growth factor-immobilized cell sheet formed in a single-layer or 3D multilayer form and a composition for inducing osteogenic differentiation including the same.

### [Background Art]

In recent years, considerable effort has been devoted to developing effective methods for treatment of tissue and organ dysfunction or treatment of organ failure. Traditional clinical approaches include a cell-based therapy in which autologous cells are transplanted or injected directly into a target site. However, difficulties associated with settling and adapting isolated cells to a target tissue have hampered practical use of such methods. Tissue engineering, in which cells and growth factors can be organized into a 3D scaffold, may offer other options. In tissues and organs, cells, the extracellular matrix, signal molecules, and the like form a complex 3D network. In such a network, cell-cell interactions and cell-extracellular matrix interactions are important in regulating biochemical and cellular responses. Tissue engineering aims to mimic biological functions without interfering with these interactions.

To achieve this goal, a biocompatible scaffold, which serves to promote cell attachment, cell proliferation, and tissue formation and acts as a structural template, is required. Generally, synthetic and natural biocompatible materials are used as extracellular matrix (ECM)-like scaffolds, which serve as a matrix to control uniform cell seeding and cell attachment, and the release of various growth factors. Recently, "scaffold-free" tissue engineering has been proposed in the field of cell sheet engineering, which may be particularly advantageous when using temperature-responsive polymers. Compared to the method of injecting isolated cells, such a scaffold-free method may increase cell attachment and proliferation, and consequently may improve integration between a cell sheet and a host tissue. In addition, the inherent functionality, structure, and integrity of EMC may be maintained. Scaffold-free tissue engineering using cell sheet technology has been applied for regeneration of damaged tissues and organs in various animal models, as well as in clinical trials for regeneration of the esophagus, cornea, and myocardium. Despite these advantages, the use of cell sheets presents several challenges. For example, to analyze the *in vitro*/*in vivo* activity of cell sheets, it is necessary to induce biochemical and cellular responses by extrinsic administration of growth factors. However, growth factors may not be sufficiently accommodated in the cells due to rapid diffusion immediately after growth factors are transferred to a target site through soluble delivery, and as a result, effective communication between cell receptors and ligands (i.e., growth factors) is difficult.

Thus, the present inventors demonstrated that polypyrrole, a conductive polymer, could be used as a highly efficient cell entrapment/release platform, and completed the present invention by developing a growth factor-immobilized cell sheet, without using a scaffold.

### [Disclosure]

### [Technical Problem]

It is an objective of the present invention to provide a cell sheet for tissue engineering and a method of preparing the same.

It is another objective of the present invention to provide a growth factor-immobilized cell sheet for tissue engineering.

It is still another objective of the present invention to provide a composition for inducing osteogenic differentiation including the growth factor-immobilized cell sheet.

### [Technical Solution]

One aspect of the present invention provides a method of preparing a cell sheet for tissue engineering, including a step of culturing target cells in a growth factor-immobilized, electroactive conductive polymer; and a step of detaching a growth factor-immobilized cell layer from an electroactive conductive polymer by applying an electric field.

According to a preferred embodiment of the present invention, the target cells may be myoblasts or mesenchymal stem cells.

According to another preferred embodiment of the present invention, the electroactive conductive polymer may be polypyrrole, a derivative thereof or an equivalent thereof, and the growth factor may be biotinylated bone morphogenetic protein 2 (BMP2), biotinylated transforming growth factor-β (TGF-β), a derivative of biotinylated BMP2, a derivative of biotinylated TGF-β, an equivalent of biotinylated BMP2 or an equivalent of biotinylated TGF-β.

According to another preferred embodiment of the present invention, the polypyrrole may be electrodeposited through biotin doping, and then using a biotin-streptavidin cross-linker, chemical conjugation of biotinylated BMP2, biotinylated TGF-β, a derivative of biotinylated BMP2, a derivative of biotinylated TGF-β, an equivalent of biotinylated BMP2 or an equivalent of biotinylated TGF-β may be achieved.

Another aspect of the present invention provides a cell sheet for tissue engineering prepared by the above-described method.

Still another aspect of the present invention provides a cell sheet for tissue engineering, which is a growth factor-immobilized cell sheet formed in a single-layer or 3D multilayer form.

According to a preferred embodiment of the present invention, the tissue engineering may be associated with treatment of tissue and organ dysfunction or treatment of organ failure.

According to another preferred embodiment of the present invention, the treatment may be for cancer patients.

According to another preferred embodiment of the present invention, the cell sheet may be used to treat any one bone disease selected from the group consisting of diseases related to bone damage, bone loss, and osteogenesis, osteitis fibrosa, adynamic bone diseases, and metabolic bone diseases or may be implanted to treat any one cartilage disease selected from the group consisting of degenerative arthritis, rheumatoid arthritis, fractures, damage to muscle tissues, plantar fasciitis, humeral lateral epicondylitis, calcific tendinitis, pseudarthrosis, and traumatic joint injuries.

Yet another aspect of the present invention provides a composition for inducing osteogenic differentiation or chondrogenic differentiation, including the above-described cell sheet for tissue engineering.

According to a preferred embodiment of the present invention, cells contained in the cell sheet may be myoblasts or mesenchymal stem cells.

### [Advantageous Effects]

The present inventors applied the inherent electroactive nature of polypyrrole to the development of novel scaffold-free cell sheet technology. The cell sheet according to the present invention is a structure that mimics a cell surface *in vivo* to which a desired ligand is bound. Therefore, the cell sheet and the method of preparing the same according to the present invention can be used for regenerative medicine and tissue engineering.

### [Description of Drawings]

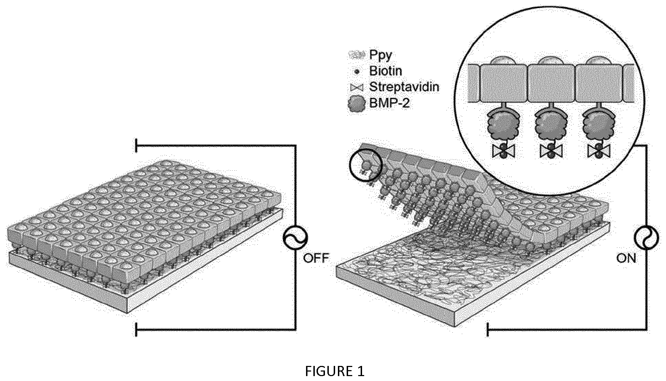

FIG. 1 illustrates preparation processes for a 3D cell sheet, on which bone morphogenetic protein 2 (BMP2) is specifically immobilized. C2C12 cells were incubated on the surface of BMP2-immobilized, biotin-doped polypyrrole. A cell sheet was prepared to be composed of individual cells to which growth factors (specifically BMP2) were effectively bound via cell surface receptors. The cell sheet was non-destructively released from the polypyrrole by applying an electric field at - 0.8V for 30 seconds. Recovered cell sheets were overlapped to have a 3D multilayer form.

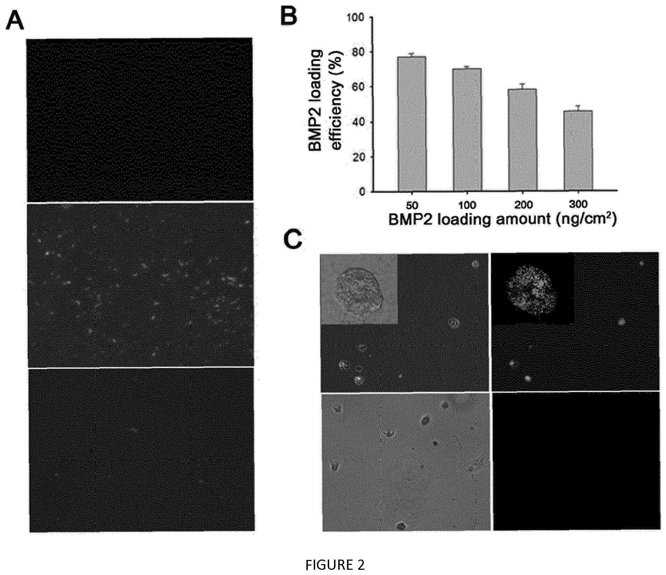

FIG. 2 (A) includes fluorescence microscope images showing polypyrrole (Ppy) alone (top), and BMP2-immobilized, biotin-doped polypyrrole before (middle) and after (bottom) electrical stimulation. When electric potential was applied at -0.8V for 30 seconds, massive release of biotin conjugated BMP2 from polypyrrole was induced. Surface-immobilized BMP2 was visualized using fluorescein isothiocyanate (FITC)-conjugated anti-BMP2 antibodies. FIG. 2 (B) is a graph showing ELISA results. Various concentrations of BMP2 were loaded, and the amount of BMP2 immobilized on the surface of biotin-doped polypyrrole was quantified. FIG. 2 (C) includes fluorescence images showing BMP2-conjugated C2C12 cells (upper panel) and normal C2C12 cells (lower panel). After electrical stimulation, detached BMP2-immobilized cells were incubated in a solution containing FITC-conjugated anti-BMP2 antibodies.

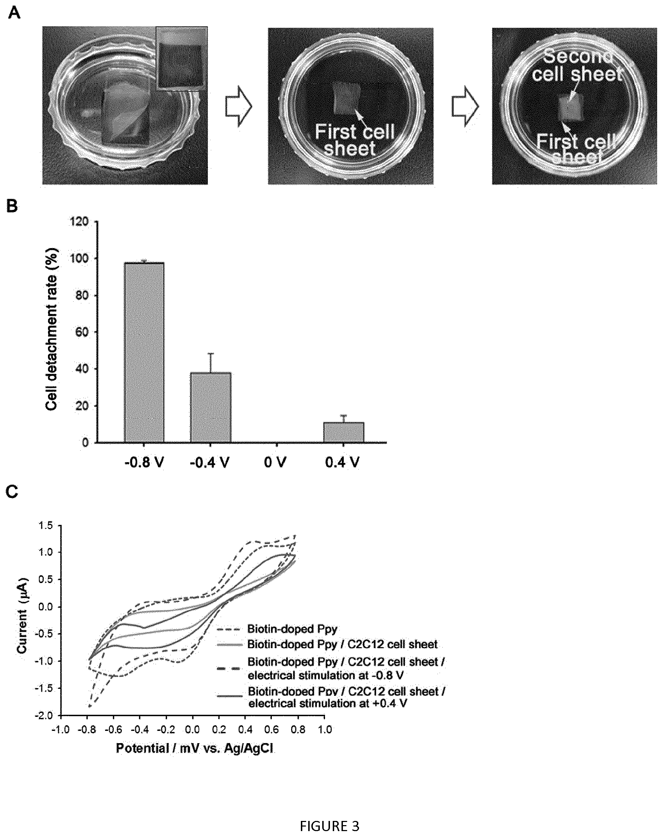

FIG. 3 (A) includes bright field images showing C2C12 cell sheet(s) detached from biotin-doped polypyrrole after applying electrical stimulation at -0.8V for 30 seconds. An embedded image shows the surface of polypyrrole after the cell sheet is detached from polypyrrole. A 3D cell sheet was prepared by repeatedly layering cell sheets detached from polypyrrole in a 35-mm cell culture dish. FIG. 3 (B) is a graph showing the efficiency of cell detachment from BMP2-immobilized, biotin-doped polypyrrole in response to an electric field (+0.4V to -0.8V for 30 seconds). FIG. 3 (C) is a graph showing the effect of electrical stimulation on cyclic voltammogram curves in a solution containing 5mM ferricyanide probes used as an indicator.

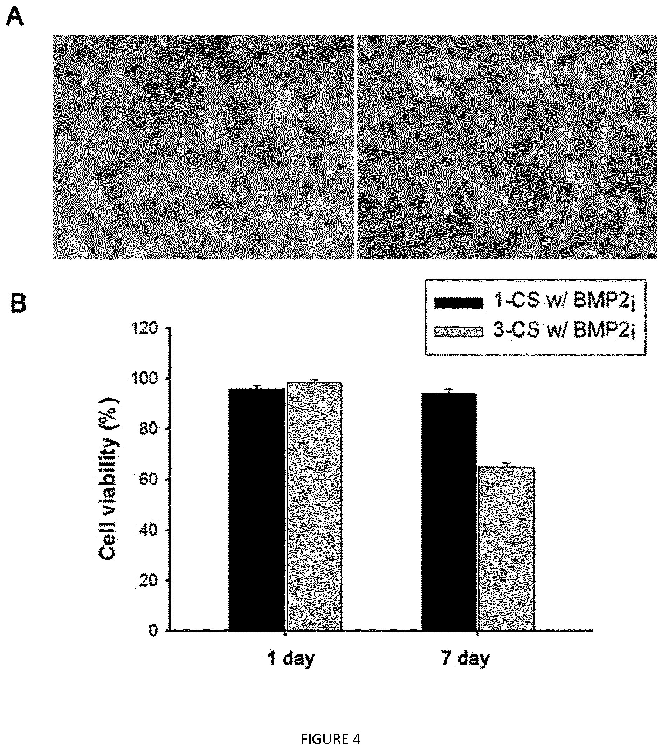

FIG. 4 (A) includes images showing the results of a fluorescence-based live/dead viability assay performed on a single-layer BMP2-immobilized C2C12 cell sheet (1-CS w/BMP2ᵢ) after one-day culture (left) or seven-day culture (right). FIG. 4 (B) is a graph comparing cell viability between a single-layer BMP2-immobilized cell sheet (1-CS w/BMP2ᵢ) and three layered, BMP2-immobilized cell sheets (3-CS w/BMP2ᵢ) after one-day culture or seven-day culture.

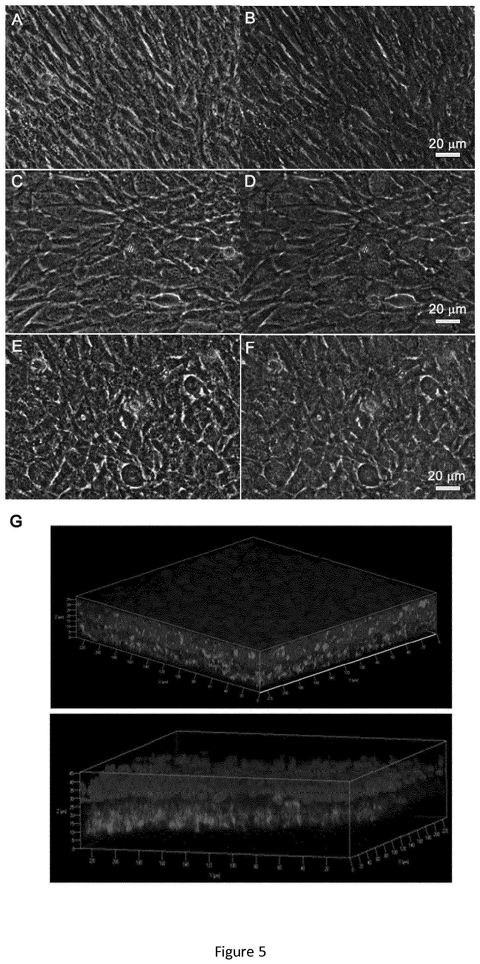

FIG. 5 includes phase contrast and fluorescence images showing (FIGS. 5A and 5B) a C2C12 cell sheet as a control, (FIGS. 5C and 5D) a C2C12 cell sheet immersed in a culture medium containing 100 ng of BMP2, and (FIGS. 5E and 5F) a C2C12 cell sheet immobilized with BMP2. After seven-day culture, electrical stimulation was applied to detach cell sheets. Fluorescence images were obtained by detecting Hoechst dye-labeled nuclei. FIG. 5 (G) includes confocal laser scanning microscopy images showing double-layer cell sheets. The first recovered cell sheet was stained with streptavidin Cy3 after cell biotinylation, and the second recovered cell sheet was stained with Hoechst dye.

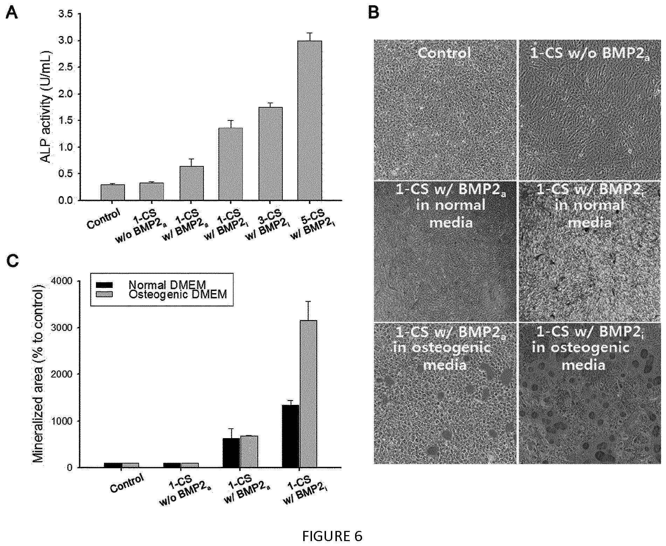

FIG. 6 (A) is a graph showing alkaline phosphatase (ALP) activity of each of a single-layer C2C12 cell sheet as a control, a single-layer C2C12 cell sheet (1-CS w/o BMP2ₐ) immersed in a culture medium not containing BMP2, a single-layer C2C12 cell sheet (1-CS w/ BMP2ₐ) immersed in a culture medium containing 100 ng of BMP2, a single-layer BMP2-immobilized C2C12 cell sheet (1-CS w/ BMP2ᵢ), three-layer BMP2-immobilized C2C12 cell sheets (3-CS w/ BMP2ᵢ), and five-layer BMP2-immobilized C2C12 cell sheets (5-CS w/ BMP2ᵢ). FIG. 6 (B) includes images showing Alizarin red staining of cell sheets incubated in normal DMEM or osteogenic DMEM for seven days. A BMP2-immobilized cell sheet (CS w/ BMP2ᵢ) was stained more darkly than a BMP2-added cell sheet (CS w/ BMP2ₐ) in both the normal medium and osteogenic medium and showed a deep red color.
FIG. 6 (C) is a graph showing the results of quantifying red colored mineralized areas using Image J software. The stained area indicates mineralization of osteoblasts.

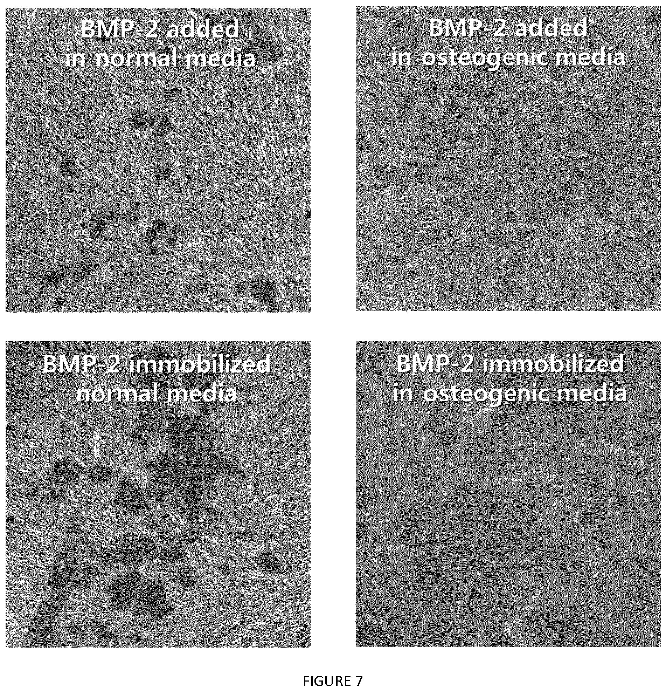

FIG. 7 includes images showing the degree of osteogenic differentiation of human mesenchymal stem cells using Alizarin red staining. Human mesenchymal stem cells were cultured in a normal medium or an osteogenic differentiation medium, and stained with Alizarin red to compare the degree of osteogenic differentiation.

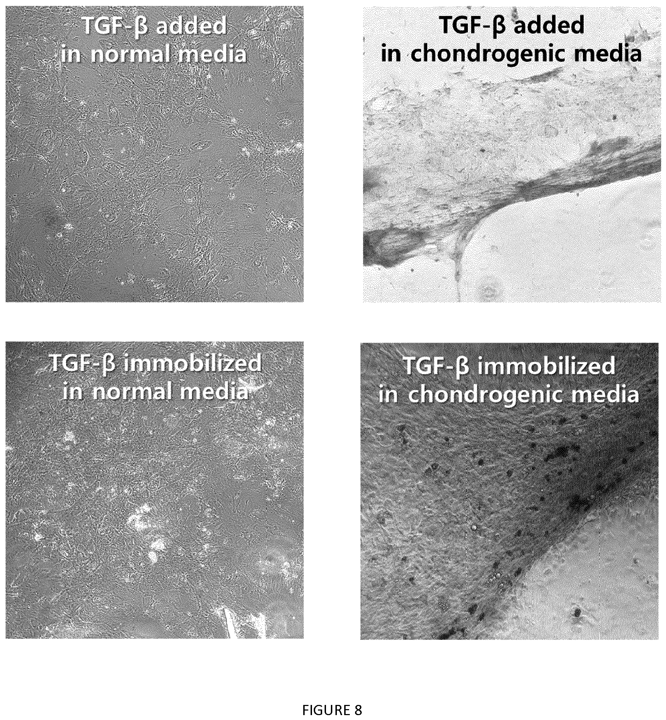

FIG. 8 includes images showing the degree of chondrogenic differentiation of human mesenchymal stem cells using Alcian blue staining. Human mesenchymal stem cells were cultured in a chondrogenic differentiation medium or a normal medium, and stained with Alcian blue to compare the degree of chondrogenic differentiation.

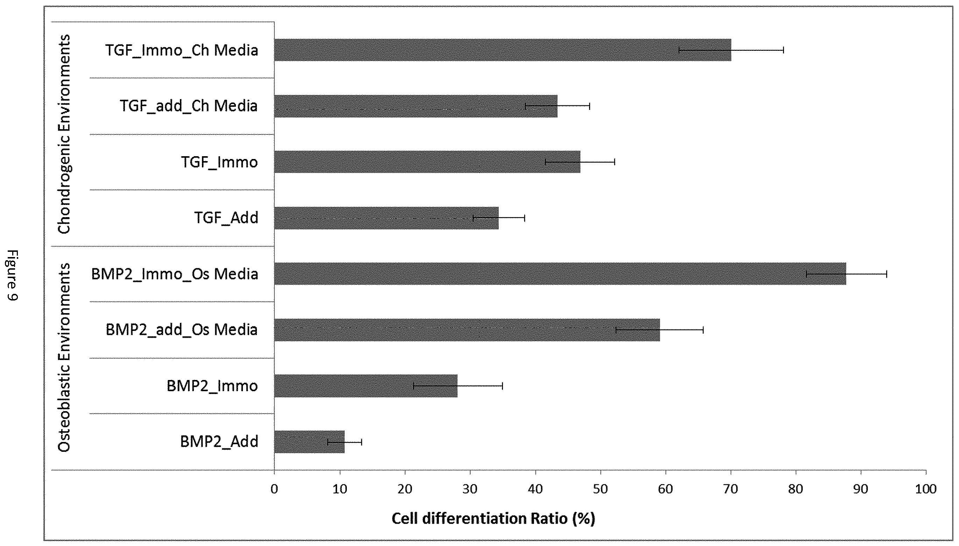

FIG. 9 is a graph illustrating the results for comparing the degree of differentiation by quantitatively analyzing the stained areas in FIGS. 7 and 8.
FIG. 10 is a schematic diagram illustrating a method of preparing 3D cell sheets.

### [Best Mode]

Hereinafter, the present invention will be described in more detail.

As described above, scaffold-free tissue engineering using cell sheet technology has been applied for regeneration of damaged tissues and organs in various animal models, as well as in clinical trials for regeneration of the esophagus, cornea, and myocardium. Despite these advantages, the use of cell sheets presents several challenges. For example, to analyze the *in vitro*/*in vivo* activity of cell sheets, it is necessary to induce biochemical and cellular responses by extrinsic administration of growth factors. However, growth factors may not be sufficiently accommodated in the cells due to rapid diffusion immediately after growth factors are transferred to a target site through soluble delivery, and as a result, effective communication between cell receptors and ligands (i.e., growth factors) is difficult.

Thus, to solve the above-described problems, in the present invention, it was demonstrated that polypyrrole, a conductive polymer, could be used as a highly efficient cell entrapment/release platform, and a growth factor-immobilized cell sheet, without using a scaffold, was developed. According to the cell sheet of the present invention, (i) biomolecules may bind to and may be released from the surface of polypyrrole by a natural and reversible redox reaction occurring at the surface of biotin-doped polypyrrole; (ii) osteogenic differentiation of C2C12 cells may be significantly improved by effective binding between receptors present in an extracellular membrane and BMP2; and (iii), since the cell sheet does not include a scaffold, the cell sheet may be further integrated with surrounding tissues, better mimicking tissue function, and may promote cell proliferation necessary for tissue regeneration. In addition, simplification of a preparation method makes it possible to prepare a scaffold-free cell sheet that may be integrated into *in vitro* tissues and organs, and the prepared cell sheets may be used for *in vivo* cell-based therapy.

The present invention provides a method of preparing a cell sheet for tissue engineering, the method including a step of culturing target cells in a growth factor-immobilized, electroactive conductive polymer; and a step of detaching a growth factor-immobilized cell layer from an electroactive conductive polymer by applying an electric field.

According to the present invention, the target cells may be myoblasts or mesenchymal stem cells. The cells may be derived from mammals such as, for example, humans, monkeys, mice, rats, dogs, cows, horses, pigs, sheep, goats, cats, rabbits, hamsters, and guinea pigs, without being limited thereto, and the cells are preferably derived from humans.

Myoblasts are undifferentiated muscle cells, and can be differentiated into bone cells by osteogenic growth factors such as BMP2.

Mesenchymal stem cells (MSCs) are multipotent stromal cells that can differentiate into a variety of cell types such as chondrocytes, bone cells, adipocytes, and muscle cells, and can be differentiated into cartilage, bone, muscle, ligaments, and adipose tissue under *in vitro* specific culture conditions. Since mesenchymal stem cells are easily extracted from bone marrow, many studies have been conducted on the possibility of using mesenchymal stem cells as a cell therapy agent for various intractable diseases. Since cartilage has low regenerative capacity, it is very difficult to treat damaged cartilage. Since degenerative arthritis is caused by degenerative changes in the joints, progression of degenerative arthritis may not be completely suppressed. So far, degenerative arthritis treatment depends on medications, physical therapy, and the like. To date, definitive drugs for treating arthritis have not yet been developed, and long-term use of steroids and lubricants results in promoting cartilage degeneration. Recently, autologous chondrocyte implantation has been developed, but there are problems such as limitation of available cartilage tissues, dedifferentiation during culturing of chondrocytes, and reduction of cell proliferation capacity due to aging. Therefore, mesenchymal stem cells having high regenerative capacity may be used as a cell therapy agent effective for regenerating cartilage in which biological restoration is lost. In addition to musculoskeletal disorders, cartilage regeneration using a cell therapy agent may be applied to digestive and urinary disorders. That is, cartilage regeneration may be applied to diseases such as reflux esophagitis and vesicoureteral reflux by locally regenerating cartilage tissue.

The number of cells seeded on an electroactive conductive polymer may be in any range as long as a cell density is sufficient to form a cell sheet. However, when a cell density is too low, cell morphology deteriorates, the incubation time required for cells to be confluent increases, and the time required for cells to mature and be colored increases. On the other hand, when cells are seeded with an excessive density, cell proliferation is inhibited, and cell death is induced due to high cell density, leading to increased incubation time required for cells to be confluent. Accordingly, a cell number to be seeded in a space having a 10 mm width and 10 mm length is 5 × 10³ to 5 × 10⁵, preferably 1 × 10⁴ to 1 × 10⁵, most preferably about 5 × 10⁴ to 1 × 10⁵.

As used herein, the term "electroactive conductive polymer" refers to an organic compound having excellent electronic, electrical, and optical properties, and at the same time, has characteristics unique to polymers such as high processability and high physical strength. In the case of a device in which an electroactive conductive polymer is applied, since electrical signals may be accurately and partially transmitted by adjusting the degree and duration of electrical stimulation, electroactive conductive polymers are attracting attention as materials used for biosensors, nerve probes, scaffolds for tissue engineering, and drug delivery systems. The electroactive conductive polymer of the present invention is not particularly limited as long as the polymer can be used as a biomaterial for medical science, and for example, may be polypyrrole, polythiophene, poly(3,4-ethylenedioxythiophene (PEDOT), polyaniline, derivatives thereof or equivalents thereof, preferably polypyrrole, a derivative thereof or an equivalent thereof.

According to the present invention, depending on the purpose of differentiation, various growth factors may be used without limitation as the growth factors. In one embodiment of the present invention, bone morphogenetic protein 2 (BMP2) was used as a growth factor for inducing osteogenic differentiation. However, in addition to BMP2, the derivatives or equivalents of BMP2 and growth factors capable of inducing osteogenic differentiation may be used without limitation. In another embodiment of the present invention, transforming growth factor-β (TGF-β) was used as a growth factor for inducing chondrogenic differentiation. However, in addition to TGF-β, the derivatives or equivalents of TGF-β and growth factors capable of inducing chondrogenic differentiation may be used without limitation. In one specific embodiment of the present invention, to stimulate osteogenesis of C2C12 myoblasts, individual cells in a C2C12 cell sheet were labeled with BMP2 (FIG. 2). In addition, to stimulate osteogenesis or cartilage formation, mesenchymal stem cells were labeled with BMP2 or TGF-β (FIGS. 7 and 8).

According to the preparation method of the present invention, when a step of detaching a growth factor-immobilized cell layer from an electroactive conductive polymer by applying an electric field is performed, an electric field having an intensity enough to non-destructively separate the cell layer from polypyrrole may be used. A cell sheet was selectively separated when a negative potential was applied. When negative potential was applied for about 30 seconds to 3 minutes at -2.0 V to -0.4 V, preferably -1.5 V to -0.8 V, the cell layer was separated with high efficiency (FIG. 3).

According to a specific embodiment of the present invention, a method of preparing a cell sheet for tissue engineering is as follows.

A conductive polymer, e.g., polypyrrole (Ppy), is electrodeposited through biotin doping, and then chemical conjugation of biotinylated BMP2 is achieved using a biotin-streptavidin cross-linker. Then, to induce interactions between cell surface receptors and the BMP2 ligands, C2C12 cells are cultured on the surface of BMP2-immobilized polypyrrole. Thereafter, by applying an electric potential, the BMP2-immobilized cell layer may be easily separated from the surface of polypyrrole (FIG. 1). This novel method results in high affinity binding between the ligand and the cell sheet, which indicates a uniform range of proteins bound to the membrane and signaling activity resulting from maximum receptor accessibility.

The preparation method may be performed by using different kinds of cells and growth factors. A preferred ligand-bound cell surface prepared using this strategy is a type of structure mimicking a biological tissue. Thus, the method of the present invention has potential applicability in regenerative medicine and tissue engineering.

In addition, the present invention provides a cell sheet for tissue engineering prepared using the above-described method.

In addition, the present invention provides a cell sheet for tissue engineering which is a growth factor-immobilized cell sheet formed in a single-layer or 3D multilayer form.

According to one embodiment of the present invention, a cell sheet prepared according to the method may be formed in a single-layer or multilayer form. According to one embodiment of the present invention, even after 1 and 7 days of culture, C1C12 cells in the prepared single-layer or multilayer cell sheets remained healthy (FIGS. 4A and 4B). In addition, in a BMP2-immobilized, single-layer cell sheet, cells had a round shape and exhibited osteogenic phenotypes after 4 days of incubation, similar to cells cultured in a culture medium supplemented with an equivalent amount of BMP2 (FIGS. 5A to 5F). A double-layer C2C12 cell sheet was formed by overlapping the first cell layer and the second cell layer, recovered by consecutive electrical stimulation, and the double-layer C2C12 cell sheet mimicked a 3D tissue (FIG. 5G).

Therefore, the single-layer or 3D multilayer cell sheet may be applied to regenerative medicine or tissue engineering, and specifically, may be usefully used in treatment of tissue and organ dysfunction or treatment of organ failure. However, there is no particular limitation on use of the cell sheet as long as the cell sheet may be effectively applied. The treatment of tissue and organ dysfunction or treatment of organ failure may include treatment for cancer patients, preferably osteosarcoma patients.

The cell sheet of the present invention may be used to prevent or treat bone diseases or cartilage diseases. "Bone disease" may be any one selected from the group consisting of diseases related to bone damage, bone loss, and osteogenesis, osteitis fibrosa, adynamic bone diseases, and metabolic bone diseases, without being limited thereto. In addition, "cartilage disease" refers to a disease caused by injuries to cartilage, cartilage tissues and/or joint tissues (synovial membranes, joint capsules, subchondral bones, and the like) by mechanical stimulation or inflammatory reactions, and may include cartilage damage diseases. Cartilage diseases may include degenerative arthritis, rheumatoid arthritis, fractures, damage to muscle tissues, plantar fasciitis, humeral lateral epicondylitis, calcific tendinitis, pseudarthrosis or traumatic joint injuries, without being limited thereto.

The cell sheet of the present invention may be used to treat the diseases in human, mammals other than humans, such as monkeys, mice, rats, dogs, horses, pigs, sheep, goats, cats, rabbits, hamsters, guinea pigs, and the like). The extent of a disease site to which the cell sheet is applicable is appropriately selected depending on the types of disease, animal species to be administered, age, sex, body weight, symptoms, and the like.

The cell sheet for implantation of the present invention may be implanted once or several times. The number of times of implantation may be determined by a healthcare practitioner or a guideline, depending on a disease. For example, in the case of performing implantation multiple times, an interval is not particularly limited, but a period of several days to several weeks may be set.

In addition, the present invention provides a composition for inducing osteogenic differentiation including the above-described cell sheet.

Cells contained in the cell sheet may be myoblasts or mesenchymal stem cells.

In one embodiment of the present invention, compared to C2C12 cells (CS w/o BMP2a) cultured in a medium without BMP2, ALP activity was clearly increased 4-fold in a single-layer BMP2-immobilized cell sheet (1-CS w/BMP2i), and ALP activity was significantly higher in a multilayer cell sheet than in a single-layer cell sheet (FIG. 6A). In addition, the Alizarin red staining results confirmed that a BMP2-immobilized cell sheet favorably influenced induction of osteogenic differentiation, and increased accumulation of mineralized calcium phosphate (FIGS. 6B and 6C).

Hereinafter, the present invention will be described in detail with reference to Examples. However, the following Examples are illustrative of the present invention, and the present invention is not limited to the following Examples.

### [Modes of the Invention]

### [Example 1] Cell sheet using myoblasts and preparation thereof

### 1. Experiments

### 1-1. Preparation of materials

Pyrrole, sodium dodecylbenzene sulfonate (NaDBS), biotin, 1-ethyl-3-(3-dimethylaminopropyl) carbodiimide (EDC), and N-hydroxysuccinimide (NHS) were purchased from Sigma-Aldrich. Recombinant human BMP2 was obtained from Peprotech.

### 1-2. Preparation of BMP2-immobilized polypyrrole (Ppy) platform

A BMP2-immobilized polypyrrole (Ppy) platform was prepared electrochemically using a potentiostat/galvanostat (BioLogic SP-50). ITO, a platinum ring, and an Ag/AgCl reference were used as a working electrode, a counter electrode, and a reference electrode, respectively. To prepare a biotin-doped Ppy platform having a surface area of 1 cm², polypyrrole was polymerized in a solution containing 0.1 M pyrrole and 0.01 M NaDBS by adding 1 mM of biotin and applying chronoamperometry (CA) at 0.8 V for 60 s. Subsequently, the biotin-doped Ppy platform was washed three times with ultrapure water, followed by air-drying. Then, 10 ng/mL of streptavidin (SA) was conjugated to biotin on the Ppy platform for 30 min and then washed with ultrapure water. Various concentrations (50, 100, 200, and 300 ng) of BMP2 were then biotinylated using a Sulfo-NHS-Biotin solution (Thermo Scientific) according to the manufacturer's protocol and added to the surface of the SA-conjugated Ppy.

### 1-3. Loading efficiency of BMP2 on polypyrrole platform

0, 100, 200 and 300 ng of BMP2-immobilized polypyrrole platforms (50, 100, 200, and 300 ng) were electrically stimulated through application of CA at -0.8 V for 30 s to induce the release of BMP2 from the surface. Supernatants were analyzed for quantifying the amount of released BMP2 using a Quantikine BMP-2 ELISA kit (R&D Systems). For visualization of BMP2 immobilized on the surface of polypyrrole, 30 µL of FITC-conjugated anti-BMP2 antibodies (5 µg/mL in PBS, Abcam) was added to the surface of the BMP2-immobilized polypyrrole and incubated at 4°C for 6 hours. After washing with PBS, 50 µL of 1 wt% bovine serum albumin dissolved in PBS was added to prevent non-specific binding. All prepared samples were observed using Zeiss Axio Observer Z1.

### 1-4. Cell culture

C2C12 myoblasts (CRL #1772) were obtained from American Type Culture Collection (ATCC) and cultured in Dulbecco's Modified Eagle's Medium (DMEM) containing 10% fetal bovine serum (FBS) and 100 units/mL antibiotics/antimycotics at 37°C under 5% CO₂. The cell culture reagents were purchased from Thermo Scientific and Life Technologies.

### 1-5. Immunofluorescence observation of BMP2 conjugated with cell surface

For immunofluorescence observation of BMP2 conjugated to C2C12 cells, C2C12 cells at a density of 1 × 10⁵ cells/mL were seeded on a BMP2-immobilized polypyrrole platform and incubated at 37°C under 5% CO₂ overnight. Then, the cells were released from the surface by exposure to an electrical field for 30 seconds, and the released cells were resuspended on a cover slip. After 3 hours, FITC-conjugated anti-BMP2 antibodies were added to the culture medium and incubated for an additional 4 hours. Labeled cells were observed using Zeiss Axio Observer Z1.

### 1-6. Cell sheet multilayering

For cell sheet multilayering, C2C12 cells were seeded at a density of 5 × 10⁴ cells on a BMP2-immobilized, biotin-doped polypyrrole (BMP2-polypyrrole) platform (10 mm width, 10 mm length) and incubated for five days in a culture medium to facilitate tight cell-cell connections. Thereafter, the polypyrrole platform was electrically stimulated with CA at -0.8V for 30 seconds. Then, a cell sheet was easily detached from the polypyrrole platform with gentle shaking and pipetting. The cell sheet was transferred to a new culture dish, and second and third cell sheets were sequentially placed on the first cell sheet according to the above-described procedures.

### 1-7. Viability of cells of cell sheet

A cell sheet recovered from a polypyrrole platform was suspended in a 12-well culture plate and maintained in a complete culture medium. To monitor the viability of cells present in the cell sheet over time, calcein AM and ethidium homodimer-1 were added to the culture medium and incubated for 20 minutes. The labeled cell sheet was observed using Zeiss Axio Observer Z1. In addition, the recovered cell sheet was suspended in a 24-well culture plate and incubated in a complete culture medium. After preparing a single-layer or three-layer cell sheet, a complete culture medium was added to each well and incubated in a 5% CO₂ incubator at 37°C. After a 24 or 48 h suspension, the viability of cells present in the cell sheet was evaluated using Cell Counting Kit-8 (Dojindo).

### 1-8. Observation of morphology of cell sheet

For morphological observation, 5 × 10⁴ cells were seeded on three types of platforms: i) biotin-doped polypyrrole, ii) biotin-doped polypyrrole immersed in a culture medium containing 100 ng/mL of BMP2, or iii) 100 ng of BMP2-immobilized polypyrrole and incubated. After seven-day culture, electrical stimulation was applied to the surface of polypyrrole to detach the cell sheet. Hoechst 33341 (Life Technologies) was added to the culture medium in which the cell sheet was included and was incubated for 30 minutes. Labeled cell sheets were observed using Zeiss Axio Observer Z1.

### 1-9. 3D observation of multilayer cell sheets

For observation of multilayer cell sheets, C2C12 cell sheets were recovered by applying electrical stimulation at -0.8 V for 30 seconds and placed in a 12-well culture plate. After cell surfaces were biotinylated using Sulfo-NHS-Biotin (0.5 mg/mL, 15 minutes), a cell sheet was stained with Hoechst 33341 (0.5 µL/mL, 1 hour), and another cell sheet was labeled with Streptavidin-Cy3 (SA-Cy3, 0.5 (µL/mL, 30 minutes). After spreading the Hoechst 33341-stained cell sheet on a cover slip, the SA-Cy3-stained cell sheet was placed on the first layer of cell sheet. The multilayer cell sheets were observed in 3D using a Zeiss LSM 710 ConfoCor 3 fluorescence microscope.

### 1-10. Alkaline phosphatase activity (ALP) assay

ALP activity was measured using an ALP assay kit (BioVision, USA) according to the manufacturer's instructions. Briefly, 5 × 10⁴ C2C12 cells were seeded and incubated on three types of platforms: i) biotin-doped polypyrrole, ii) biotin-doped polypyrrole immersed in a culture medium containing 100 ng/mL of BMP2, or iii) a 100 ng of a BMP2-immobilized polypyrrole platform. After five day-incubation, all groups of cell sheets were recovered by electrical stimulation and prepared into one-, three-, and five-layer sheets. Then, the cell sheets were transplanted into a 6-well culture plate and incubated for seven days. Thereafter, cell sheets were lysed using an ALP assay buffer for 1 hour, and cell lysates were centrifuged at 13,500 rpm, at 4°C for 10 minutes. After centrifugation, supernatants were placed in a 96-well plate, p-nitrophenyl phosphate was added to each well, and the mixtures were incubated at 37°C for 30 minutes. Alkaline phosphatase activity was measured in the samples using a microplate reader (Power Wave HT, BioTek) at a wavelength of 405 nm.

### 1-11. Alizarin red staining

Alizarin red staining was performed on the prepared cell sheets according to a standard protocol. C2C12 cells at a density of 5 × 10⁴ were incubated on a biotin-doped polypyrrole platform or a BMP-2 loaded polypyrrole platform to form tight cell-cell connection. Then, cells were recovered from the surface of polypyrrole by applying electrical stimulation at -0.8 V for 30 seconds and transplanted into a 6-well culture plate. After 24 hours, the culture medium was exchanged with DMEM containing 10% FBS or DMEM containing 2 mM L-glutamine, 50 µM ascorbic acid, 20 mM β-glycerol phosphate, and 10% FBS. Then, cell sheets were incubated for another seven days in an incubator set to 37°C with 5% CO₂. Thereafter, the cell sheets were washed twice with deionized water and stained using 40 mM Alizarin red (Sigma-Aldrich, pH 4.2 adjusted with 1% of ammonium hydroxide) solution for 20 minutes at room temperature. Finally, cells were washed with deionized water and observed under a microscope. The calcium deposited area observed in the obtained microscope image was analyzed using Image J software (NIH, USA).

### 2. Results

### 2-1. C2C12 cell sheet specifically conjugates to BMP 2

FIG. 1 is a schematic diagram showing preparation processes for 3D cell sheets. First, polypyrrole was electrochemically polymerized on the surface of ITO by using biotin as the co-dopant of a polypyrrole film. Biotin can act as a bridge to connect target biomolecules. Using this approach, electric-field-assisted cell sheets were prepared, and a mouse skeletal muscle-derived C2C12 cell line was used as a working model. Importantly, a growth factor, specifically bone morphogenetic protein 2 (BMP2), was efficiently conjugated to individual cells present in a 3D construct via cell surface receptors. BMP2 plays an important role in inducing osteoblastic differentiation of C2C12 myoblasts by blocking the myogenic differentiation pathway. When BMP2 is introduced near cell surfaces, in addition to communication between cell membrane receptors and BMP2, the recognition of cell membrane receptors for BMP2 may be increased. As a result, complexes between BMP2 and receptors are stably formed, leading to sustained receptor activation. This strategy allows manipulation of individual target cells with the desired functional entity, and furthermore, the modulation of cellular activity. The advantages of the polypyrrole-based scaffold-free cell sheet of the present invention are as follows: (i) biomolecules may bind to and may be released from the surface of polypyrrole by a natural and reversible redox reaction occurring at the surface of biotin-doped polypyrrole; (ii) osteogenic differentiation of C2C12 cells may be significantly improved by effective binding between receptors present in an extracellular membrane and BMP2 (osteogenic differentiation of C2C12 cells may be achieved by efficient and site-specific delivery of functional proteins); (iii) Since the cell sheet does not include a scaffold, the cell sheet may be further integrated with surrounding tissue, better mimicking tissue function, and may promote cell proliferation necessary for tissue regeneration. In addition, the simplification of a preparation method makes it possible to prepare a scaffold-free cell sheet that may be integrated into *in vitro* tissues and organs, and the prepared cell sheets may be used for *in vivo* cell-based therapy.

### 2-2. Loading efficiency of BMP2 on polypyrrole platform

The feasibility of this approach was investigated by examining the immobilization efficiency of biotinylated BMP2 on the surface of a functionalized polypyrrole. In general, when C2C12 cells are cultured in a medium containing BMP2, since BMP2 is delivered to target cells through soluble delivery, there is limited interaction between BMP2 and cell surface receptors. To overcome this problem, a cell sheet, in which individual cells are conjugated with BMP2, was prepared. First, the binding efficiency of BMP2 to biotin-doped polypyrrole was analyzed using fluorescence microscopy; by analyzing fluorescence images, fluorescence images were used to examine the distribution of surface-immobilized proteins having FITC-conjugated anti-BMP2 antibodies as a detection probe. The green fluorescence observed on the surface of polypyrrole indicated the presence of BMP2 due to the preferential association of the biotin-streptavidin linkage. However, fluorescent signals emitted from the surface of polypyrrole disappeared, in response to an applied electric field, which may primarily be explained by the massive release of biotin and conjugated BMP2 from polypyrrole. BMP2 loading efficacy was examined using various concentrations of BMP2. As shown in FIG. 2B, when 50 to 300 ng/cm² of BMP2 was applied to the surface of biotin-doped polypyrrole, the amount of immobilized BMP2 (quantified by ELISA) was 39 to 147 ng/cm², suggesting that the level of surface-immobilized BMP2 would be sufficient to enhance availability thereof to cell surface receptors. In addition, the presence of BMP2 on the surface of C2C12 cells was demonstrated (FIG. 2C). By applying an electric field, cells that were specifically bound to growth factors via membrane receptors were released. Then, BMP2-conjugated C2C12 cells were incubated in a solution containing FITC-conjugated anti-BMP2 antibodies. Finally, BMP2 was observed along the cell membranes of BMP2-conjugated C2C12 cells but not in normal C2C12 cells (FIG. 2C). In the present invention, it was demonstrated that individual cells within a cell sheet could be labeled with BMP2 to stimulate osteogenesis in C2C12 myogenic cells. Biotin within the surface of polypyrrole allows greater flexibility for the incorporation of new biological moieties, which may be a versatile and molecularly well-defined methodology for cell surface engineering. In addition, biotin-containing polypyrrole may be used for a variety of cellular applications.

### 2-3. Preparation of BMP2-immobilized C2C12 cell sheet using biotin-doped polypyrrole platform

As shown in FIG. 3, the structure of a BMP2-conjugated C2C12 cell sheet was determined. First, C2C12 cells (5 × 10⁴) were seeded on the surface of BMP2-immobilized, biotin-doped polypyrrole and incubated for 5 days to promote tight contact between cells. Cells assembled in the form of a sheet were non-destructively detached from the surface of polypyrrole by applying an electric field (FIG. 3A). In fact, a weak electric potential delicately modulated the unique cell-surface interface by causing the spontaneous release of biotin moieties and attached cells from the surface. Recovered cell sheets were easily prepared as a 3D multilayer. The effect of electric potentials on manipulating cell sheet technology was investigated by applying an electrical field ranging from -0.8 V to +0.4 V to the surface of polypyrrole for 30 seconds (FIG. 3B). Consistent with previous studies, a single-layer cell sheet was readily detached from the surface by gentle agitation using a PBS-filled pipette after a negative electrical stimulation was applied. However, when a positive electrical stimulation was applied, no reaction was observed on the surface of polypyrrole, which was similar to that of a non-stimulated control. As shown in FIG. 3C, the electrochemical behavior of the surface of polypyrrole was evaluated using cyclic voltammetry (CV). Redox peak currents were enhanced on the surface of biotin-doped polypyrrole. However, C2C12 cell sheets grown on the surface of BMP2-immobilized, biotin-doped polypyrrole exhibited obviously decreased electron-transfer capability, specifically of electroactive ferricyanide species throughout the surface of polypyrrole, due to tight cell-surface junctions. Electrical stimulation at -0.8 V was sufficient to detach a cell sheet from the surface of polypyrrole, which could ultimately restore the current intensity to an original state thereof by allowing the free transfer of electrolytes. However, a positive electric potential did not affect peak intensity, which indicates that C2C12 cell sheets remained firmly attached to the surface of polypyrrole regardless of the applied electrical stimulation. Electrical stimulation induces a conformational change in polypyrrole backbones via oxidation/reduction reactions; specifically, a polypyrrole polymer swells significantly with a positive potential, generating free volume and enabling entrapment of various moieties inside a polymeric backbone. In contrast, at negative potentials, polypyrrole undergoes structural shrinkage and squeezes out molecules incorporated within the polymer. Indeed, these results demonstrated preferential detachment of cell sheets only when negative electrical potentials were applied.

### 2-4. Characterization of BMP2-immobilized C2C12 cell sheet

After 1 days and 7 days of incubation, the viability of single-layer or multilayer cell sheet(s) was measured. C2C12 cells remained healthy even after 7 days (FIGS. 4A and 4B). In addition, the morphology of cells of a single-layer cell sheet was examined using a phase contrast and fluorescence microscopy (FIGS. 5A to 5F). In a BMP2-immobilized cell sheet, cells had a round shape and exhibited osteogenic phenotypes after 4 days of incubation, similar to cells cultured in a culture medium supplemented with an equivalent amount of BMP2. In contrast, cells cultured in a conventional culture dish without BMP2 exhibited a normal spindle-shaped morphology. Confocal laser scanning microscopy was used to observe a bilayer composed of C2C12 cells. After sequential electrical stimulation, the recovered second cell layer overlapped with the first cell layer, thereby mimicking 3D tissue formation. The prepared cell sheets had a thickness of approximately 35 µm. The effect of BMP2-immobilized C2C12 cell sheets on osteoblastic expression was examined. After electrical stimulation, BMP2-immobilized cell sheets (CS w/BMP2ᵢ) were transferred to a culture dish to assess alkaline phosphatase (ALP) activity in the C2C12 cells (FIG. 6A). The ALP activity of a single-layer BMP2-immobilized cell sheet (1-CS w/BMP2ᵢ) was apparently increased 4-fold compared with that of C2C12 cells cultured in a BMP2-free medium (CS w/o BMP2ₐ). In additional, ALP activity was significantly higher in multilayer sheets than single-layer sheets, which indicated that 3D tissues created by stacking individual cell sheets could be more therapeutically effective when transplanted. In comparison with cell sheets cultured in a BMP2-added medium (CS w/BMP2ₐ), enhanced ALP activity in CS w/BMP2ᵢ could be attributed to membrane-bound growth factors, achieved through receptor-ligand complex formation. Growth factor-tethered cell sheets showed efficient cellular activity, which significantly correlated with the defined amount of BMP2 present in individual cells, which would be unlikely in traditional soluble delivery methods. In fact, direct binding of biomolecules to individual cells yields uniform distribution and long-term contact duration by restricting diffusion from the integration site, and thereby efficiently triggers differentiation. Osteoblastic differentiation of C2C12 cells within 3D cell constructs was examined using an Alizarin red staining assay (FIGS. 6B to 6C). Interestingly, CS w/BMP2; showed more intense red staining than CS w/BMP2ain both a normal cell medium and an osteogenic medium. The Alizarin red staining results showed that a BMP2-immobilized cell sheet favorably influenced induction of osteogenic differentiation, and increased accumulation of mineralized calcium phosphate. CS w/BMP2ᵢ induced a 4-fold increase in mineral deposition compared to CS w/BMP2ₐ, especially in an osteogenic medium.

### 3. Conclusions

The present inventors developed a BMP2-immobilized C2C12 cell sheet without using an artificial scaffold. The surface of electroactive, biotin-doped polypyrrole of the cell sheet was capable of conjugating with BMP2 at biologically relevant levels via a biotin-streptavidin interaction. After using ELISA to quantify immobilized BMP2 on the surface of polypyrrole, C2C12 cells were incubated on polypyrrole to (i) strengthen the cell-cell junction and (ii) modify cell surface receptors with BMP2 ligands. After electrical stimulation with a negative potential, BMP2-bound cell sheets were non-destructively detached and transferred to culture dishes, where osteogenic differentiation capabilities thereof were assessed. The above-described results indicate that this approach could be a valuable and flexible tool for attaching bioactive molecules to cell surfaces in applications that require cell culture, tissue engineering, or both.

### [Example 2] Cell sheet using mesenchymal stem cells and preparation thereof

### 1. Experiments

A 0.01M PSS solution (hereinafter, referred as 'pyrrole solution') containing 0.1 M pyrrole and 1 mM biotin, sulfo-NHS-biotin, TGF-β (chondrogenic differentiation factor) as a cell growth (differentiation) factor, and BMP2 (osteocyte differentiation factor) were used to prepare a human mesenchymal stem cell (ATCC, PCS-500-011) sheet. The preparation method is as follows:
① To electrically polymerize a polypyrrole film (Ppy film), ITO glass having a size of 2 × 1.5 cm is dipped in a pyrrole solution, and chronoamperometry (CA) is applied at 0.8 V for 60 seconds. At this time, the film is prepared so that the surface size of polypyrrole is 1 cm².
② After washing twice with deionized water, a solution containing 30 mM EDC and 6 mM NHS is applied to activate biotin on the surface of polypyrrole (incubation conditions: at room temperature (RT) for 30 minutes).
③ After washing twice with deionized water, a solution containing streptavidin at a concentration of 10 ng/mL is applied to label the surface of polypyrrole with streptavidin (incubation conditions: at RT for 30 minutes).
④ Wash twice with deionized water.
⑤ A solution containing 1 mM sulfo-NHS-biotin is applied to the surface of the polypyrrole film, followed by incubation at RT for 30 minutes.
⑥ After washing twice with deionized water, TGF-β and BMP-2, each at a concentration of 10 ng/mL, are applied to the surface of the polypyrrole film, followed by incubation at 4°C for 1 hour.
⑦ After washing the surface of the polypyrrole film with a cell culture medium, human mesenchymal stem cells at a density of 1 × 10⁵ are suspended on the surface of the polypyrrole film and incubated in an incubator set at 37 °C with 5 % CO₂ for 4 hours so that the cells are immobilized on the surface of the polypyrrole film.
⑧ After confirming that the cells are spread on the surface of the polypyrrole film, the ITO glass is transferred to each well of a 6-well plate, and then a normal medium or a differentiation medium is added to each well, followed by incubation for 14 days. The media are changed every 3 days. The medium supplemented with BMP-2 and TGF-β, each at a concentration of 10 ng/mL, is also changed every 3 days.
⑨ After 14 days, cells in each well are electrically stimulated under the conditions of CA at -1.5 V for 3 minutes to implant a cell sheet into a normal culture plate.
⑩ After 24 hours, cells are stained with Alizarin red and Alcian blue, respectively, to compare the degree of differentiation.

### 2. Results

After detaching a cell sheet from the surface of a polypyrrole film using electrical stimulation, the cell sheet was stained with Alizarin red and Alcian blue, respectively, to compare the degree of differentiation between cells grown in a medium containing a growth factor and cells grown on the surface of polypyrrole on which a growth factor was immobilized.

As a result, as shown in FIG. 7, more calcium deposition occurred in an experimental group cultured on the surface of a BMP-2 immobilized polypyrrole film, compared to a control group cultured in a BMP-2 supplemented medium. A similar pattern was also observed in an experimental group cultured in an osteogenic differentiation medium

In addition, as shown in FIG. 8, the degree of differentiation into chondrocytes was higher in an experimental group cultured on the surface of a TGF-β immobilized polypyrrole film than in a control group cultured in a TGF-β supplemented medium. A similar pattern was also observed in an experimental group cultured in a chondrogenic differentiation medium.

FIG. 9 is a graph comparing the degree of differentiation by quantitatively analyzing the portions stained in FIGS. 7 and 8. These results confirmed that a method of culturing cells by immobilizing a growth factor or a differentiation factor in a limited space such as a polypyrrole film is more effective than a method of culturing cells in a culture medium supplemented with a growth factor or a differentiation factor.

### [Industrial Applicability]

The cell sheet of the present invention has a cell surface on which a desired ligand is immobilized, thus mimicking a biological tissue. Thus, the cell sheet and the method of preparing the same according to the present invention can be used in regenerative medicine and tissue engineering.

## Claims

1. A method of preparing a cell sheet for tissue engineering, the method comprising:
a step of culturing target cells on a growth factor-immobilized, electroactive conductive polymer; and
a step of detaching a growth factor-immobilized cell layer from the electroactive conductive polymer by applying an electric field.

2. The method according to claim 1, wherein the target cells are myoblasts or mesenchymal stem cells.

3. The method according to claim 1, wherein the electroactive conductive polymer is polypyrrole, a derivative thereof or an equivalent thereof, and the growth factor is biotinylated bone morphogenetic protein 2 (BMP2), biotinylated transforming growth factor-β (TGF-β), a derivative of biotinylated BMP2, a derivative of biotinylated TGF-β, an equivalent of biotinylated BMP2 or an equivalent of biotinylated TGF-β.

4. The method according to claim 3, wherein the polypyrrole is electrodeposited through biotin doping, and then using a biotin-streptavidin cross-linker, chemical conjugation of biotinylated BMP2, biotinylated TGF-β, a derivative of biotinylated BMP2, a derivative of biotinylated TGF-β, an equivalent of biotinylated BMP2 or an equivalent of biotinylated TGF-β is achieved.

5. A cell sheet for tissue engineering prepared by the method according to any one of claims 1 to 4.

6. A cell sheet for tissue engineering, which is a growth factor-immobilized cell sheet is formed in a single-layer or 3D multilayer form.

7. The cell sheet according to claim 5 or 6, wherein the tissue engineering is associated with treatment of tissue and organ dysfunction or treatment of organ failure.

8. The cell sheet according to claim 7, wherein the treatment is for cancer patients.

9. The cell sheet according to claim 5 or 6, wherein the cell sheet is used to treat any one bone disease selected from the group consisting of diseases related to bone damage, bone loss, and osteogenesis, osteitis fibrosa, adynamic bone diseases, and metabolic bone diseases or is implanted to treat any one cartilage disease selected from the group consisting of degenerative arthritis, rheumatoid arthritis, fractures, damage to muscle tissues, plantar fasciitis, humeral lateral epicondylitis, calcific tendinitis, pseudarthrosis, and traumatic joint injuries.

10. A composition for inducing osteogenic differentiation or chondrogenic differentiation, comprising the cell sheet according to claim 5 or 6.

11. The composition according to claim 10, wherein cells contained in the cell sheet are myoblasts or mesenchymal stem cells.
